# EUROPEAN PATENT SPECIFICATION

(11) **EP 2 621 407 B1**
(45) Date of publication and mention of the grant of the patent: **17.01.2018**
(21) Application number: 11770003.9
(22) Date of filing: 30.09.2011
(51) Int. Cl.: A61F 2/24

(54) **INTRA-ANNULAR MOUNTING FRAME FOR AORTIC VALVE REPAIR**
RINGINTERNER MONTAGERAHMEN FÜR AORTENKLAPPENREPARATUR
CADRE DE MONTAGE INTRA-ANNULAIRE POUR RÉPARATION DE VALVES AORTIQUES

(30) Priority: 30.09.2010 US 388573 P
(43) Date of publication of application: 07.08.2013
(73) Proprietor: Biostable Science & Engineering, Inc., Austin, TX 78754 (US)
(72) Inventor: RANKIN, J., Scott, Nashville TN 37205 (US); BEAVAN, Ai, Kingsland TX 78639 (US)
(74) Representative: Jones, Nicholas Andrew
(86) International application number: PCT/US2011/054160
(87) International publication number: WO 2012/044901

(56) References cited:
- WO-A1-97/16135
- US-A- 3 714 671
- US-A- 5 607 471
- US-A1- 2008 086 204
- US-A1- 2008 097 593

## Description

### BACKGROUND OF THE INVENTION

### 1. FIELD OF THE INVENTION

The present disclosure relates to an elliptical mounting frame useful for applications including aortic valve repair. More particularly, the present disclosure relates to an elliptical intra-annular mounting frame that is designed for insertion directly into the aortic valve annulus. The disclosure also includes methods for the insertion and implantation of the elliptical intra-annular mounting frame, as well as complementary devices, such as ascending aortic DACRON® grafts and pericardial single or multiple cusp prostheses.

### 2. DESCRIPTION OF THE RELATED ART

The mammalian heart is essentially a pump that functions as a chemo-mechanical energy transducer. The chemical energy of metabolic substrates and oxygen is converted into the mechanical energy of blood pressure and flow by myocardial sarcomeres during cardiac contraction. The pump is periodic at a frequency of 1-2 Hz, with the contraction/ejection phase called systole and the relaxation/filling phase termed diastole.

The human heart is the center of the cardiovascular system, the system having two parallel circulations consisting of the pulmonary circulation and the systemic circulation. The pulmonary circulation receives blood from the vena cava into the right atrium and right ventricle, and then pumps the cardiac output into the pulmonary arteries and through the lungs. The systemic circulation receives blood from the pulmonary veins, pumps the cardiac output through the left atrium and left ventricle to the aorta, systemic arteries, capillaries, and veins, and finally transmits blood back to the vena cava. The mitral valve is positioned between the upper chamber, the left atrium, and the pumping chamber, the left ventricle. The left atrium acts in a capacitor function receiving blood from the lungs via the pulmonary veins throughout the cardiac cycle. The left ventricle fills during diastole by receiving blood from the left atrium as the mitral valve opens, and then during systole, the mitral valve closes and permits forward ejection of the blood from the left ventricle into the ascending aorta. The aortic valve is located between the left ventricle and aorta, and functions under normal conditions to allow unimpeded blood flow out of the ventricle and into the aorta during systole. During diastole, the aortic valve closes and prevents regurgitation backward into the left ventricle.

Surgical reconstruction of a patient's native valve is becoming standard for mitral valve disease. Whether considering mitral valve prolapse, pure annular dilatation, ischemic mitral regurgitation, or mitral endocarditis, repair is now routine, highly successful, and associated with low late failure rates. Even in rheumatic mitral disease, many surgeons are embarking on programs of aggressive repair, adding to ring annuloplasty the techniques of posterior leaflet augmentation with gluteraldehyde-fixed autologous pericardium, resection of the stenotic submitral apparatus with insertion of artificial GORE-TEX® chords, leaflet decalcification, *etc.* The current goal is to achieve close to a 100% repair rate of mitral valve disorders and to markedly diminish prosthetic valve replacement. The advantages of repair versus replacement in this setting are well documented. The operative mortality rate (normalized for other factors) is lower, anticoagulation is not required in sinus rhythm, valve-related complications are less than with prosthetic valves, durability is excellent because the patent's own tissues do not degenerate, and late endocarditis is reduced because less foreign material is present. As such, these concepts for mitral valve disease are rapidly becoming standard-of-care in the field of cardiac surgery.

The aortic valve of a human heart can also become diseased, with aortic valve insufficiency occurring from a number of causes. A common cause is annular dilatation, with the sinuses of the Valsalva migrating outward and the inter-commissural distances expanding. Geometrically, this derangement not only increases the annular circumference, but also reduces the surface area of cusp coaptation. The coaptation angle of the cusps is changed essentially from being parallel and meeting at an acute angle to pointing at each other, wherein the cusps comprise a more obtuse arrangement. Eventually, a central gap of coaptation occurs and increasing aortic insufficiency begets more annular dilatation, which begets more aortic insufficiency and the leak progressively increases.

Repair of a diseased aortic valve has not met with the same success experienced in reconstructing a diseased mitral valve. For about 10 to 15 years, the "commissural annuloplasty" technique has been used, but it can only be applied to mild-to-moderate secondary aortic insufficiency, usually in patients undergoing primary coronary bypass or mitral valve procedures. Commissural annuloplasty not only decreases annular circumference, but also tends to move the sinuses centrally, thus normalizing geometry and coaptation angles of the cusps. There is a limit, however, to the geometric abnormality that commissural annuloplasty can normalize, and because the entire annulus is not fixed by this procedure, the potential for further dilatation and recurrent aortic insufficiency exists.

Unfortunately, previously known supra- or infra-annular rings and artificial valves are generally not effective for the long term improvement of the aortic valve, and additionally, may require quite complicated surgical procedures. The rings currently described for insertion into the aorta are designed to be inserted above or below the valve. Suturing a ring below the aortic valve (infra-annular) to simply downsize or constrict the circumference will negatively distort the valve cusps and can lead to worsening valve leak. Furthermore, the constriction concept ignores the fact that the three semi-lunar aortic valve cusps are three-dimensional structures that are required to meet in space in a specific orientation to provide valve competence. Similarly, previously disclosed supra-annular rings are laden with the same problems, and have even less geometric basis, since the supra-annular rings only quite roughly follow the shape of the aortic tissue above the annulus.

What is desired, therefore, is an improved device for aortic valve repair. Also desired is a process for inserting and mounting such devices.

### BRIEF SUMMARY OF THE INVENTION

The present disclosure provides intra-annular mounting frames that are roughly elliptical in shape and have posts that flare outward from a vertical plane passing through the opening of the frame, and methods of implanting such intra-annular mounting frames. The disclosed intra-annular mounting frames provide improved results in the repair of defective aortic valves. Prior art devices known from US 3714671, US 5607471 and US 2008/0086204 work on similar principles to the device of the invention but do not have the claimed geometry. The present disclosure provides an intra-annular mounting frame for an aortic valve comprising a plurality of curvatures having ends, a plurality of points interconnecting the ends of each of the curvatures to form an interior area, and a plurality of posts extending upward from each of the points, wherein the points, the ends of the curvatures, and the posts define edge regions that flare outward from the interior area of the intra-annular mounting frame, and wherein the intra-annular mounting frame has an elliptical shape having a major axis and a minor axis, wherein the major axis is greater in length than the minor axis. In certain embodiments the intra-annular mounting frame comprises three curvatures, three points, and three posts. A circumferential distance (distance around the perimeter of the ellipse) is defined between each of the posts in the intra-annular mounting frame. In some embodiments, generally depending upon the specific geometry of the aortic valve of the patient, the circumferential distance between each of the posts are equal (symmetrical), in other embodiments the circumferential distance between each of the posts are different (asymmetrical), while in yet other embodiments two of the circumferential distances between the posts are equivalent while the third circumferential distance is different from the other two (also asymmetrical).

In further embodiments the intra-annular mounting frame comprises two curvatures, two points, and two posts. Such embodiments are used in bicuspid aortic valve repair. In certain embodiments, generally depending upon the specific geometry of the bicuspid aortic valve of the patient, the circumferential distance (distance around the perimeter of the ellipse) between each of the posts are equal (symmetrical), while in other embodiments the circumferential distance between each of the posts are different (asymmetrical). In additional embodiments the posts are located along the curvatures defined by the major axis of the ellipse, the curvatures defined by the minor axis of the ellipse, or one post is located along the curvatures defined by the major axis of the ellipse while the other post is located along the curvatures defined by the minor axis of the ellipse.

Also defined by the intra-annular mounting frame is an angle between the edge regions, defined as the region including the ends of the curvatures, the points and the posts, and the vertical plane of the intra-annular mounting frame. In certain aspects, generally depending upon the specific geometry of the aortic valve of the patient, the edge regions of the intra-annular mounting frame flare outward from a vertical plane passing through the interior area of the intra-annular mounting frame at an angle of about 5 degrees, about 6 degrees, about 7 degrees, about 8 degrees, about 9 degrees, about 10 degrees, about 11 degrees, about 12 degrees, about 13 degrees, about 14 degrees, about 15 degrees. In some embodiments the curvatures and/or posts comprise a plurality of perforations or stripes.

In particular embodiments, again generally depending upon the specific geometry of the aortic valve of the patient, the ratio of the major axis of the ellipse to the minor axis of the ellipse is between about 1.1 and 1.8, including ratios of about 1.1, about 1.2, about 1.3, about 1.4, about 1.5, about 1.6, about 1.7, and about 1.8. The ellipse defined by the intra-annular mounting frame can also be expressed as the ratio of the minor axis to the major axis. As such, the presently described intra-annular mounting frame can have a ratio of the minor axis to the major axis of less than 1, in certain aspects about 0.9, about 0.85, about 0.80, about 0.75, about 0.70, about 0.65, about 0.60, or about 0.55 or so. In various embodiments the length of the major axis of the ellipse is between about 10 millimeters and about 35 millimeters, between about 15 millimeters and about 30 millimeters, between about 20 millimeters and about 25 millimeters, between about 10 millimeters and about 30 millimeters, between about 10 millimeters and about 25 millimeters, between about 10 millimeters and about 20 millimeters, between about 10 millimeters and about 15 millimeters, between about 15 millimeters and about 35 millimeters, between about 20 millimeters and about 35 millimeters, between about 25 millimeters and about 35 millimeters, or between about 30 millimeters and about 35 millimeters, including lengths of about 10 millimeters, about 11 millimeters, about 12 millimeters, about 13 millimeters, about 14 millimeters, about 15 millimeters, about 16 millimeters, about 17 millimeters, about 18 millimeters, about 19 millimeters, about 20 millimeters, about 21 millimeters, about 22 millimeters, about 23 millimeters, about 24 millimeters, about 25 millimeters, about 26 millimeters, about 27 millimeters, about 28 millimeters, about 29 millimeters, about 30 millimeters, about 31 millimeters, about 32 millimeters, about 33 millimeters, about 34 millimeters, and about 35 millimeters. The length of the minor axis of the ellipse can also vary, for example between about 8 millimeters and about 25 millimeters, between about 10 millimeters and about 21 millimeters, between about 14 millimeters and about 18 millimeters, between about 8 millimeters and about 20 millimeters, between about 8 millimeters and about 15 millimeters, between about 10 millimeters and about 25 millimeters, between about 15 millimeters and about 25 millimeters, or between about 20 millimeters and about 25 millimeters, including lengths of about 8 millimeters, about 9 millimeters, about 10 millimeters, about 11 millimeters, about 12 millimeters, about 13 millimeters, about 14 millimeters, about 15 millimeters, about 16 millimeters, about 17 millimeters, about 18 millimeters, about 19 millimeters, about 20 millimeters, about 21 millimeters, about 22 millimeters, about 23 millimeters, about 24 millimeters, and about 25 millimeters.

In certain embodiments the intra-annular mounting frame is comprised of a plastic, a polymer, a metal, a thermoplastic, a resin, or any combination thereof, or other materials that allow for slight deformation but will not sheer under normal stresses. In other embodiments the intra-annular mounting frame is coated or covered. Such coatings or coverings include, but are not limited to, a polymer-type fiber cloth, gluteraldehyde-fixed bovine or human pericardium, or various combinations of the available coatings and coverings known to those of skill in the art.

Thus, the present disclosure also provides a method of repairing an aortic valve having an aortic wall, cusps and commissures, comprising providing an intra-annular mounting frame comprising a plurality of curvatures having ends, a plurality of points interconnecting the ends of each of the curvatures to form an interior area, and a plurality of posts extending upward from each of the points, wherein the points, the ends of the curvatures, and the posts define edge regions that flare outward from the interior area of the intra-annular mounting frame, and wherein the intra-annular mounting frame has an elliptical shape having a major axis and a minor axis, inserting the intra-annular mounting frame directly into the aortic valve annulus with the intra-annular mounting frame positioned up under the cusps, and suturing the intra-annular mounting frame into the aortic valve with the sutures passing through the aortic wall and passing both above and below the cusps, thereby repairing the aortic valve. In certain aspects the sutures, for example mattress sutures, pass through perforations or stripes in the intra-annular mounting frame. In additional aspects, the method further comprises buttressing tissue of the aortic wall by placing pledgets, for example TEFLON® felt or DACRON® pledgets, onto the sutures above the cusps of the aortic valve.

The present disclosure additionally provides a method of repairing an aortic valve having an aortic wall, cusps and commissures, comprising the steps of providing an intra-annular mounting frame comprising a plurality of curvatures having ends, a plurality of points interconnecting the ends of each of the curvatures to form an interior area, and a plurality of posts extending upward from each of the points, wherein the points, the ends of the curvatures, and the posts define edge regions that flare outward from the interior area of the intra-annular mounting frame, and wherein the intra-annular mounting frame has an elliptical shape having a major axis and a minor axis, wherein the major axis is greater in length than the minor axis, inserting the intra-annular mounting frame directly into the aortic valve annulus with the intra-annular mounting frame positioned up under the cusps, employing a plurality of support arcs similar in shape to the curvatures of the intra-annular mounting frame above the cusps of the aortic valve, and passing sutures through the aortic wall and between the intra-annular mounting frame and the plurality of support arcs, thereby repairing the aortic valve. In certain aspects the plurality of support arcs are cloth covered arcs.

Throughout this disclosure, unless the context dictates otherwise, the word "comprise" or variations such as "comprises" or "comprising," is understood to mean "includes, but is not limited to" such that other elements that are not explicitly mentioned may also be included. Further, unless the context dictates otherwise, use of the term "a" may mean a singular object or element, or it may mean a plurality, or one or more of such objects or elements. Additionally, in discussions of the elliptical shape of the devices, the terms "circumference" and "perimeter" are used interchangeably.

### BRIEF DESCRIPTION OF THE SEVERAL VIEWS OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****.** Perspective view of one embodiment of an elliptical intra-annular mounting frame of the present disclosure.
**FIG. 2****.** Front elevational view of the embodiment of the elliptical intra-annular mounting frame shown in **FIG. 1****.**
**FIG. 3****.** Top view of the embodiment of the elliptical intra-annular mounting frame shown in **FIG. 1****.**
**FIG. 4****.** Side view from "A" (see **FIG. 3**) of the embodiment of the elliptical intra-annular mounting frame shown in **FIG. 1****.**
**FIG. 5****.** Perspective view of one embodiment of an asymmetrical elliptical intra-annular mounting frame of the present disclosure.
**FIG. 6****.** Front elevational view of the embodiment of the asymmetrical elliptical intra-annular mounting frame shown in **FIG. 5****.**
**FIG. 7****.** Top view of the embodiment of the asymmetrical elliptical intra-annular mounting frame shown in **FIG. 1****.**
**FIG. 8****.** Side view from "A" (see **FIG. 7**) of the embodiment of the asymmetrical elliptical intra-annular mounting frame shown in **FIG. 1****.**
**FIG. 9****.** Perspective view of one embodiment of a bicuspid elliptical intra-annular mounting frame of the present disclosure.
**FIG. 10****.** Front elevational view of the embodiment of the bicuspid elliptical intra-annular mounting frame shown in **FIG. 9****.**
**FIG. 11****.** Top view of the embodiment of the bicuspid elliptical intra-annular mounting frame shown in **FIG. 9****.**
**FIG. 12****.** Side view from "D" (see **FIG. 11**) of the embodiment of the bicuspid elliptical intra-annular mounting frame shown in **FIG. 9****.**

### DETAILED DESCRIPTION OF THE INVENTION

The present disclosure arises at least in part from the discovery by the inventors that the annulus of the aortic valve is not circular in shape, as generally believed in the art, but is actually elliptical in shape, and that the commissures of the aortic valve flare outward from the center of the valve. Therefore an intra-annular mounting frame that is roughly elliptical in shape, and that has outwardly flaring commissures, will provide improved results in the repair of defective aortic valves.

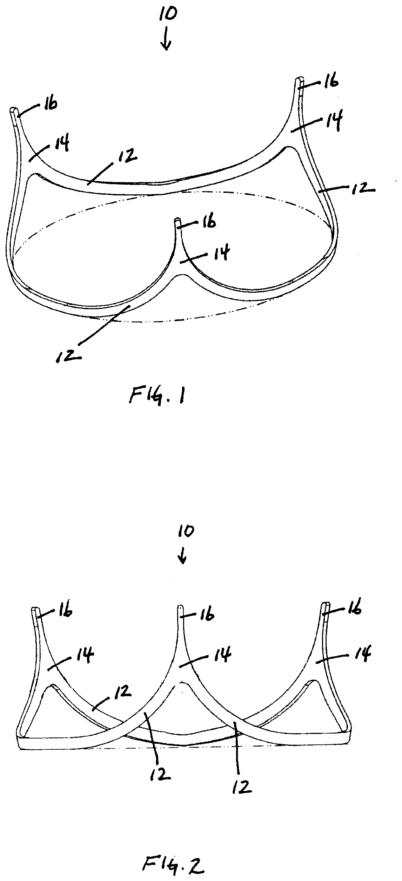

Referring now to **FIG. 1**, a perspective view of an embodiment of an elliptical intra-annular mounting frame useful for aortic valve repair is shown and generally designated as numeral **10.** Elliptical intra-annular mounting frame **10** is inserted into the aortic valve annulus and provides for the reconstruction of the native aortic valve.

Elliptical intra-annular mounting frame **10** includes a plurality of curvatures **12,** interconnecting points **14,** and posts **16.** Generally, curvatures **12** conform to the annular cusp geometry with interconnecting points **14** and posts **16** conforming to the geometry of the sub-commissural region. Curvatures **12** curve in a plurality of planes of elliptical intra-annular mounting frame **10** to correspond to the three-dimensional geometry of the cusps of an aortic valve. For reference, the horizontal plane is defined as the plane on which the elliptical intra-annular mounting frame **10** would rest with each curvature **12** contacting the plane. The vertical plane is defined as the plane which intersects the horizontal plane at a perpendicular angle and runs vertically through the elliptical intra-annular mounting frame **10.** Curvatures **12** may curve in both the horizontal and vertical planes, and/or may curve in one or more other planes. Generally curvatures **12** contact the aortic wall and provide support and alignment to the aortic valve cusps. Interconnecting points **14** and posts **16** provide support to the commissures of the aortic valve. Specifically, interconnecting points **14** and posts **16** are designed to closely fit the three-dimensional geometry between adjacent cusps and locate near the commissures thus providing support and assistance in the restoration of the proper coaptation of the cusps. Interconnecting points **14** continuously narrow to posts **16** and thus fit within the narrowing space between adjacent cusps that culminates in a commissure. As such, interconnecting points **14** and posts **16** provide support within this inter-cusp space to immediately below the commissures.

Referring now to **FIG. 2****,** a front elevational view of elliptical intra-annular mounting frame **10** of **FIG. 1** is shown. Once again elliptical intra-annular mounting frame **10** includes a plurality of curvatures **12,** interconnecting points **14,** and posts **16.**

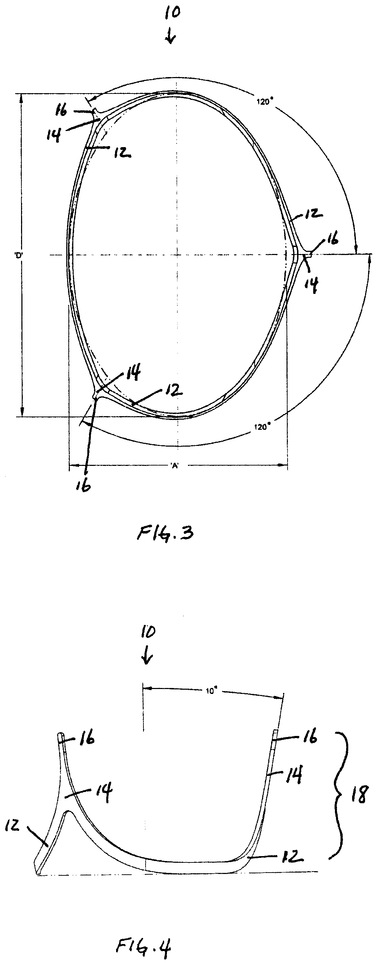

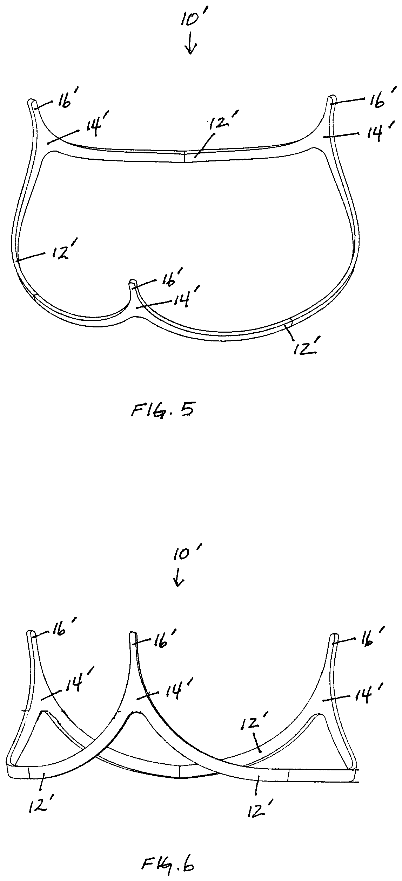

Referring now to **FIG. 3**, a top view of elliptical intra-annular mounting frame **10** is shown. Once again elliptical intra-annular mounting frame **10** includes a plurality of curvatures **12,** interconnecting points **14,** and posts **16.** As shown in **FIG. 3****,** the base of elliptical intra-annular mounting frame **10** generally defines an ellipse, with a major axis denoted by "D" and a minor axis denoted by "A". In the embodiment of the elliptical intra-annular mounting frame **10** shown in **FIG. 3****,** the ratio of the major axis to the minor axis of the ellipse is about 1.5:1, although in other embodiments of the elliptical intra-annular mounting frame (not shown) the ratio of the major axis to the minor axis of the ellipse can vary generally between about 1.7:1 or 1.8:1 and about 1.1:1 or 1.2:1. In addition, the circumferential distances (distances around the perimeter of the ellipse) between posts **16** in the embodiment of the elliptical intra-annular mounting frame **10** shown in **FIG. 3** are roughly equivalent (symmetric; about 33% of the circumference or perimeter), although in other embodiments of the elliptical intra-annular mounting frame (see, for example, **FIG. 5**) the circumferential distances between posts **16** can differ, for example two of the circumferential distances between posts **16** can be roughly equivalent while the third circumferential distance can differ from the other two, or all three circumferential distances can be different from each other, depending on the specific geometry of the aortic valve to be repaired. Thus, asymmetric aortic valve geometries can be repaired using the presently described elliptical intra-annular mounting frame.

Referring now to **FIG. 4**, a side view of elliptical intra-annular mounting frame **10** is shown. Once again elliptical intra-annular mounting frame **10** includes a plurality of curvatures **12,** interconnecting points **14,** and posts **16.** In the embodiment of the elliptical intra-annular mounting frame **10** shown in **FIG. 4****,** the three edge portions **18** of the intra-annular mounting frame **10** that comprise interconnecting points **14,** posts **16,** and upper portions of two curvatures **12** flare outward from the vertical plane of the elliptical intra-annular mounting frame by about 10°. However, in other embodiments of the elliptical intra-annular mounting frame (not shown) the three edge portions **18** can flare outward from the vertical plane of the elliptical intra-annular mounting frame by between about 1° or so and about 30° or so, with the flare between 5 and 15 degrees falling under the scope of the claims. Additionally, although in the embodiment of the elliptical intra-annular mounting frame shown in **FIG. 4** the three edge portions **18** each flare outward at equal angles from the vertical plane, in further embodiments (not shown) the three edge portions **18** can flare outward from the vertical plane at different angles, for example two of the edge portions **18** can flare outward at equal angles from the vertical plane while the third edge portion **18** can flare outward from the vertical plane at a different angle than the other two edge portions **18,** or all three edge portions **18** can flare outward at different angles from the vertical plane, depending on the specific geometry of the aortic valve to be repaired.

Different orientations and shapes of the curvatures may be utilized to account for different anatomic variations of the aortic valve. As detailed above in **FIG. 1** through **FIG. 4****,** in most embodiments the curvatures are fairly symmetrical to one another, as most aortic valves have 3 cusps of equal sizes. However, in additional embodiments the elliptical intra-annular mounting frame can be produced in an asymmetrical design as some patients have aortic valves with asymmetrical sinuses. Variations could include an elliptical intra-annular mounting frame with one curvature length about 20% larger than the other two curvature lengths, a variation with a single curvature length sized 20% smaller than the other curvature lengths, or variations with each of the curvature lengths having a different size. Additionally, since some patients have an aortic valve that has only two cusps (bicuspid), a bicuspid elliptical intra-annular mounting frame can be produced with two curvatures and two interconnecting points. Examples of such asymmetric and bicuspid elliptical intra-annular mounting frames are detailed below.

Referring now to **FIG. 5****,** a perspective view of an embodiment of an asymmetrical elliptical intra-annular mounting frame useful for aortic valve repair is shown and generally designated as numeral **10'.** Asymmetrical elliptical intra-annular mounting frame **10**' is inserted into the aortic valve annulus and provides for the reconstruction of the native aortic valve having asymmetrical sinuses. Asymmetrical elliptical intra-annular mounting frame **10'** includes a plurality of curvatures **12',** interconnecting points **14',** and posts **16'.** As in the case of the symmetrical elliptical intra-annular mounting frame **10** detailed in **FIG. 1** through **FIG. 4****,** above, curvatures **12**' conform to the annular cusp geometry with interconnecting points **14'** and posts **16'** conforming to the geometry of the sub-commissural region. Curvatures **12'** curve in a plurality of planes of asymmetrical elliptical intra-annular mounting frame **10'** to correspond to the three-dimensional geometry of the cusps of an asymmetric aortic valve. For reference, the horizontal plane is defined as the plane on which asymmetrical elliptical intra-annular mounting frame **10'** would rest with each curvature **12'** contacting the plane. The vertical plane is defined as the plane which intersects the horizontal plane at a perpendicular angle and runs vertically through asymmetrical elliptical intra-annular mounting frame **10'.** Curvatures **12**' may curve in both the horizontal and vertical planes, and/or may curve in one or more other planes. Generally curvatures **12**' contact the aortic wall and provide support and alignment to the aortic valve cusps. Interconnecting points **14'** and posts **16'** provide support to the commissures of the aortic valve. Specifically, interconnecting points **14'** and posts **16'** are designed to closely fit the three-dimensional geometry between adjacent cusps and locate near the commissures, thus providing support and assistance in the restoration of the proper coaptation of the cusps. Interconnecting points **14'** continuously narrow to posts **16'** and thus fit within the narrowing space between adjacent cusps that culminates in a commissure. As such, interconnecting points **14'** and posts **16'** provide support within this inter-cusp space to immediately below the commissures.

Referring now to **FIG. 6****,** a front elevational view of asymmetrical elliptical intra-annular mounting frame **10'** of **FIG. 5** is shown. Once again asymmetrical elliptical intra-annular mounting frame **10**' includes a plurality of curvatures **12',** interconnecting points **14',** and posts **16'.**

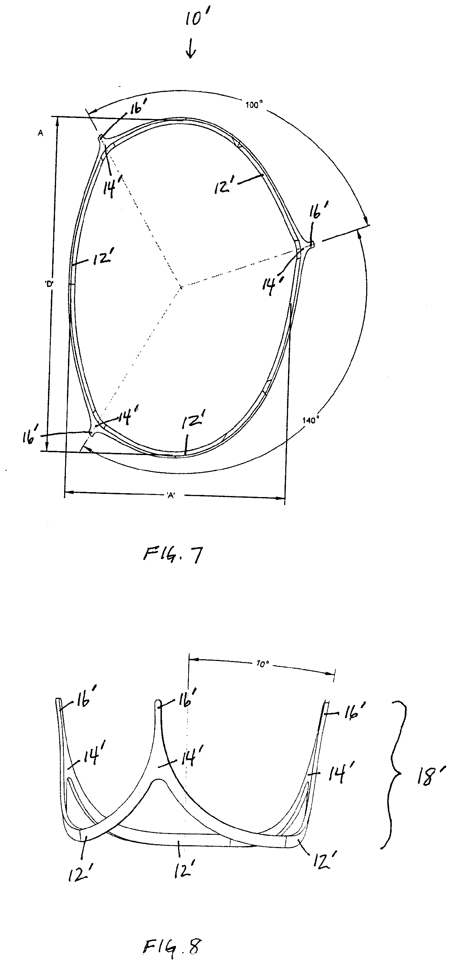

Referring now to **FIG. 7**, a top view of asymmetrical elliptical intra-annular mounting frame **10'** is shown. Once again asymmetrical elliptical intra-annular mounting frame **10'** includes a plurality of curvatures **12',** interconnecting points **14',** and posts **16'.** As shown in **FIG. 7**, the base of asymmetrical elliptical intra-annular mounting frame **10**' generally defines an ellipse, with a major axis denoted by "D" and a minor axis denoted by "A". In the embodiment of the asymmetrical elliptical intra-annular mounting frame **10'** shown in **FIG. 7****,** the ratio of the major axis to the minor axis of the ellipse is about 1.5:1, although in other embodiments of the elliptical intra-annular mounting frame (not shown) the ratio of the major axis to the minor axis of the ellipse can vary generally between about 1.7:1 or 1.8:1 and about 1.1:1 or 1.2:1. In addition, the circumferential distances (distances around the perimeter of the ellipse) between posts **16'** in the embodiment of the asymmetrical elliptical intra-annular mounting frame **10'** shown in **FIG. 7** are each different (roughly 28%, 33% and 39% of the circumference), although in other embodiments of the asymmetrical elliptical intra-annular mounting frame (not shown) two of the circumferential distances between posts **16'** can be roughly equivalent while the third circumferential distance can differ from the other two, depending on the specific geometry of the asymmetric aortic valve to be repaired. Thus, all asymmetric aortic valve geometries can be repaired using the presently described elliptical intra-annular mounting frame.

Referring now to **FIG. 8****,** a side view of asymmetrical elliptical intra-annular mounting frame **10'** is shown. Once again asymmetrical elliptical intra-annular mounting frame **10'** includes a plurality of curvatures **12',** interconnecting points **14',** and posts **16'.** In the embodiment of the asymmetrical elliptical intra-annular mounting frame **10**' shown in **FIG. 8****,** the three edge portions **18'** of the asymmetrical elliptical intra-annular mounting frame **10'** that comprise interconnecting points **14',** posts **16',** and upper portions of the curvatures **12'** flare outward from the vertical plane of the elliptical intra-annular mounting frame by about 10°. However, in other embodiments of the asymmetrical intra-annular mounting frame (not shown) the three edge portions **18'** can flare outward from the vertical plane of the elliptical intra-annular mounting frame by between about 1° or so and about 30° or so. Additionally, although in the embodiment of the asymmetrical elliptical intra-annular mounting frame **10'** shown in **FIG. 8** the three edge portions **18'** each flare outward at equal angles from the vertical plane (about 10°), in additional embodiments (not shown) the three edge portions **18'** can flare outward from the vertical plane at different angles, for example two of the edge portions **18'** can flare outward at equal angles from the vertical plane while the third edge portion **18'** can flare outward from the vertical plane at a different angle than the other two edge portions **18',** or all three edge portions **18'** can flare outward at different angles from the vertical plane, depending on the specific geometry of the asymmetric aortic valve to be repaired.

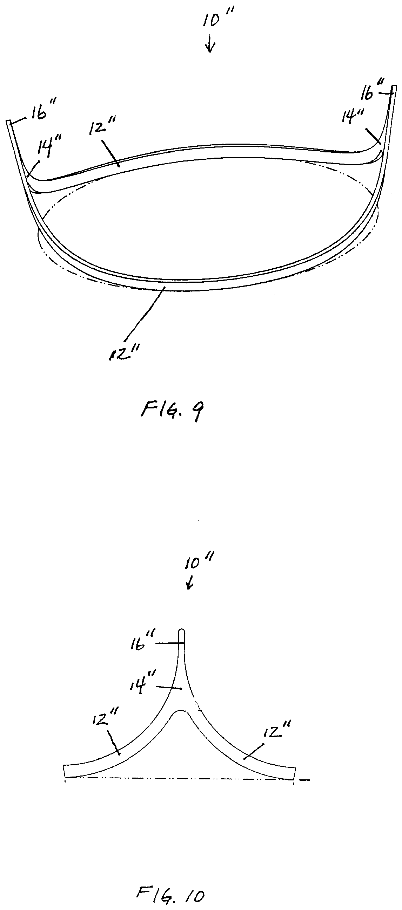

Referring now to **FIG. 9****,** a perspective view of an embodiment of a bicuspid elliptical intra-annular mounting frame useful for aortic valve repair is shown and generally designated as numeral **10".** Bicuspid elliptical intra-annular mounting frame **10"** is inserted into the aortic valve annulus and provides for the reconstruction of the native aortic valve having only two sinuses. Bicuspid elliptical intra-annular mounting frame **10"** includes two curvatures **12",** interconnecting points **14",** and posts **16".** As in the case of the symmetrical elliptical intra-annular mounting frame **10** detailed in **FIG. 1** through **FIG. 4****,** above, curvatures **12"** conform to the annular cusp geometry with interconnecting points **14"** and posts **16"** conforming to the geometry of the sub-commissural region. Curvatures **12"** curve in a plurality of planes of bicuspid elliptical intra-annular mounting frame **10"** to correspond to the three-dimensional geometry of the two cusps of a bicuspid aortic valve. For reference, the horizontal plane is defined as the plane on which bicuspid elliptical intra-annular mounting frame **10"** would rest with each curvature **12"** contacting the plane. The vertical plane is defined as the plane which intersects the horizontal plane at a perpendicular angle and runs vertically through bicuspid elliptical intra-annular mounting frame **10".** Curvatures **12"** may curve in both the horizontal and vertical planes, and/or may curve in one or more other planes. Generally curvatures **12"** contact the aortic wall and provide support and alignment to the aortic valve cusps. Interconnecting points **14"** and posts **16"** provide support to the commissures of the aortic valve. Specifically, interconnecting points **14"** and posts **16"** are designed to closely fit the three-dimensional geometry between adjacent cusps and locate near the commissures thus providing support and assistance in the restoration of the proper coaptation of the cusps. Interconnecting points **14"** continuously narrow to posts **16"** and thus fit within the narrowing space between adjacent cusps that culminates in a commissure. As such, interconnecting points **14"** and posts **16"** provide support within this inter-cusp space to immediately below the commissures.

Referring now to **FIG. 10****,** a front elevational view of bicuspid elliptical intra-annular mounting frame **10"** is shown. Bicuspid elliptical intra-annular mounting frame **10"** includes two curvatures **12",** interconnecting points **14",** and posts **16".**

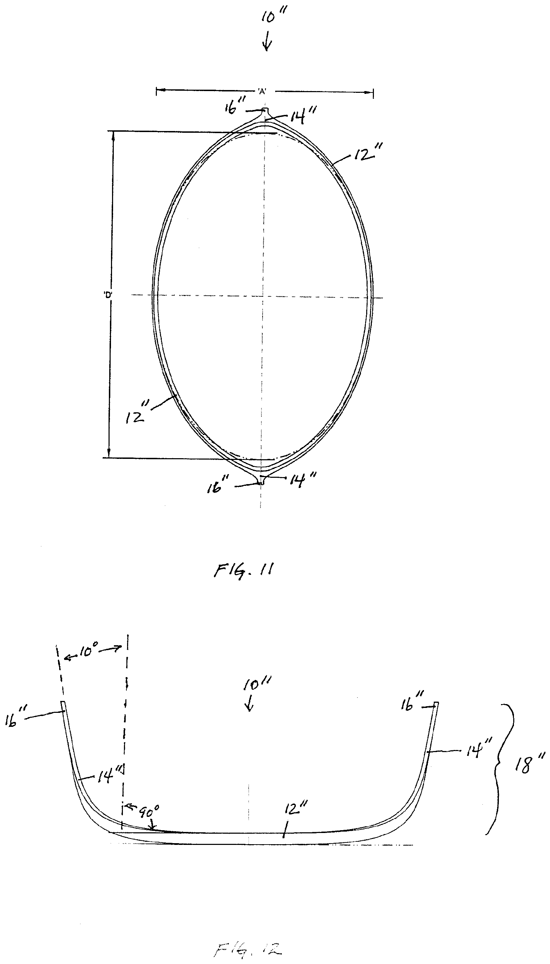

Referring now to **FIG. 11****,** a top view of bicuspid elliptical intra-annular mounting frame **10"** is shown. Once again bicuspid elliptical intra-annular mounting frame **10"** includes two curvatures **12",** interconnecting points **14",** and posts **16".** As shown in **FIG. 11****,** the base of bicuspid elliptical intra-annular mounting frame **10"** generally defines an ellipse, with a major axis denoted by "D" and a minor axis denoted by "A". In the embodiment of the bicuspid elliptical intra-annular mounting frame **10"** shown in **FIG. 11****,** the ratio of the major axis to the minor axis of the ellipse is about 1.5:1, although in other embodiments of the bicuspid elliptical intra-annular mounting frame (not shown) the ratio of the major axis to the minor axis of the ellipse can vary generally between about 1.7:1 or 1.8:1 and about 1.1:1 or 1.2:1. In addition, the circumferential distances (distances around the perimeter of the ellipse) between posts **16"** in the embodiment of the bicuspid elliptical intra-annular mounting frame **10"** shown in **FIG. 11** are roughly equivalent (symmetric; about 50% of the circumference), although in other embodiments of the bicuspid elliptical intra-annular mounting frame (not shown) the circumferential distances between posts **16"** can differ, for example one circumferential distance of about 75% of the circumference and the other circumferential distance of about 25% of the circumference, one circumferential distance of about 70% of the circumference and the other circumferential distance of about 30% of the circumference, one circumferential distance of about 65% of the circumference and the other circumferential distance of about 35% of the circumference, one circumferential distance of about 60% of the circumference and the other circumferential distance of about 40% of the circumference, one circumferential distance of about 55% of the circumference and the other circumferential distance of about 45% of the circumference, or the like, depending on the specific geometry of the bicuspid aortic valve to be repaired. Thus, all asymmetric bicuspid aortic valve geometries can be repaired using the presently described elliptical intra-annular mounting frame.

Referring now to **FIG. 12****,** a side view of bicuspid elliptical intra-annular mounting frame **10"** is shown. Once again bicuspid elliptical intra-annular mounting frame **10"** includes two curvatures **12",** interconnecting points **14",** and posts **16".** In the embodiment of the bicuspid elliptical intra-annular mounting frame **10"** shown in **FIG. 12****,** the two edge portions **18"** of the bicuspid intra-annular mounting frame **10"** that comprise interconnecting points **14",** posts **16",** and upper portions of the two curvatures **12"** flare outward from the vertical plane of the bicuspid elliptical intra-annular mounting frame by about 10°. However, in other examples of the bicuspid elliptical intra-annular mounting frame (not shown) the two edge portions **18"** can flare outward from the vertical plane of the intra-annular mounting frame by between about 1° or so and about 30° or so, with the embodiments of the invention encompassing a flare from 5° to 15°. Although in the embodiment of the bicuspid elliptical intra-annular mounting frame **10"** shown in **FIG. 12** the two edge portions **18"** each flare outward at equal angles from the vertical plane, in additional embodiments (not shown) the two edge portions **18"** can flare outward from the vertical plane at different angles, depending on the specific geometry of the bicuspid aortic valve to be repaired.

Most generally, the major axis of the elliptical intra-annular mounting frame is from about 10 millimeters to about 35 millimeters or so in length, and the minor axis of the elliptical intra-annular mounting frame is from about 8 millimeters to about 25 millimeters or so in length, with a variety of different sized frames there between, forming a gradient of possible choices to closely approximate the needs of the patient. However, larger sizes of the elliptical intra-annular mounting frame can be produced to be utilized with patients that have aortic root aneurysms or Marfan's syndrome. Furthermore, the elliptical intra-annular mounting frame height as measured from the base of a curvature to the tip of a post may vary, but generally ranges from about 8 millimeters to about 15 millimeters or so.

The elliptical intra-annular mounting frame can be comprised of metal, plastics, thermoplastics, polymers, resins or other materials that will remain intact in spite of potentially high tension caused from a highly dilated aortic root. The elliptical intra-annular mounting frame may be constructed of a solid metal wire, solid plastic, or a perforated strip of metal or plastic so as to provide the sutures better purchase once implanted into the aortic valve. The perforations may vary depending on the installation method, though with the generally uniform geometry of the annular region, a set number and position of perforations for sutures may be created and marked onto the elliptical intra-annular mounting frame. In certain embodiments the elliptical intra-annular mounting frame can include a GORE-TEX® coating. In further embodiments, the elliptical intra-annular mounting frame can be covered or coated with a variety of polymers or polymer resins, including, but not limited to, polyethylene terephthalate, sold under the name DACRON® cloth. Alternatively, the elliptical intra-annular mounting frame can be covered with gluteraldehyde-fixed bovine pericardium, which can be useful in certain embodiments as high blood velocities in the outflow tract of the left ventricle could possibly predispose the patient to hemolysis with a cloth covering.

One of the many advantages of the elliptical intra-annular mounting frame is the ease in which the required frame size can be determined preoperatively. Imaging techniques such as Magnetic Resonance Imaging (MRI), echocardiography, or computer tomography (CT)-angiography can be used non-invasively to determine the measurements required to prepare an elliptical intra-annular mounting frame for the patient. In further embodiments the imaging device, including an MRI machine or CT-angiography machine and related controls, could include system parameters and mathematical models and descriptions of the elliptical intra-annular mounting frame that automatically takes the measurements of the patient's aortic valve and outputs the appropriately sized elliptical intra-annular mounting frame required to restore competency of the patient's aortic valve. Additional data output could include the display of various sized elliptical intra-annular mounting frames for restoring competency and the effect each different frame would create upon implantation.

In certain embodiments, the presently described elliptical intra-annular mounting frames may have perforations on the curvatures and posts for the passage of sutures therethrough. For example, the sutures may be horizontal mattress sutures, which may then pass into the aortic wall beneath the aortic valve annulus. In one particular arrangement, the sutures could pass deep into the aortic wall, under the cusps, allowing for the insertion of an elliptical intra-annular mounting frame directly into an aortic valve annulus, which would closely correspond to the cusps and commissures. Optionally, three horizontal mattress sutures may be utilized per cusp and one per commissure with a total of twelve sutures used to implant the elliptical intra-annular mounting frame. One of skill in the art would understand that less or more sutures, as well as other attachment techniques known in the art, may be utilized to position and attach the elliptical intra-annular mounting frame into the aortic valve annulus. Above the aortic valve, pledgets may be placed onto the mattress sutures to preclude the possible tearing of aortic tissue. The pledgets may be TEFLON® felt pledgets, or in other embodiments pieces or strips of fabric such as DACRON® may be utilized with the mattress sutures rather than pledgets. The pledgets would generally be small so they would not interfere with the mobility of the aortic valve leaflets.

In an alternative embodiment for installing an elliptical intra-annular mounting frame, support arcs may be employed above the valve annulus, into which sutures could be inserted. Such support arcs may comprise three curvatures with a shape that is substantially similar to those of the elliptical intra-annular mounting frame, which corresponds to the curvature and geometry of the attachment of the cusps to the aortic wall as well as the commissures, resulting in the annulus of the aortic valve being "sandwiched" between the elliptical intra-annular mounting frame and support arcs. Sutures may extend through perforations in the elliptical intra-annular mounting frame through the aortic wall to the support arcs above the cusps, attaching also through perforations in the support arcs. In additional embodiments, the sutures may extend around the support arcs or attach using other methods known in the art.

The described elliptical intra-annular mounting frame and related methods of sizing and implanting the elliptical intra-annular mounting frame could also be applied to other pathologies. With aortic root aneurysms, the elliptical intra-annular mounting frame could allow leaflet-sparing root replacement to be performed from inside the aorta, without the need for extensive external dissection, as with current procedures. A non-porous DACRON® graft may be utilized with the elliptical intra-annular mounting frame after being scalloped and flared in the graft's proximal aspect, to conform to the sinuses of Valsalva. The size of the graft may be selected to match the size of the elliptical intra-annular mounting frame, with consideration also being given for the diameter of the distal aorta. The coronary arteries could then be anastomosed to the side of the graft, either as buttons or with the inclusion technique. Using this simple method, the aortic valve annulus would be fixed in size and geometry, the native aortic valve would be repaired and preserved, and the entire root and ascending aorta could be replaced for root aneurismal disease, with much less dissection and difficulty than with current techniques.

Other pathologies also could be addressed using the described elliptical intra-annular mounting frame. Ultrasonic debridement could be used adjunctively to remove spicules of calcium, and portions of leaflets could be resected and replaced with gluteraldehyde-fixed autologous pericardium. This concept also includes the option of aortic valve single or multiple cusp replacement. With a method of fixing root geometry through reorientation, and potentially undersizing it slightly, more complex repairs could be undertaken, with the elliptical intra-annular mounting frame compensating for slight imperfections. If one cusp were severely diseased or prolapsing, for example, the cusp could be replaced with a gluteraldehyde-fixed autologous (or in certain instances bovine) pericardial cusp (of the appropriate size and geometry to match the size of the elliptical intra-annular mounting frame and native cusps). The artificial cusp could be attached to the arc above the elliptical intra-annular mounting frame, with the elliptical intra-annular mounting frame acting as an attachment for the arc and artificial leaflet. The patient's other valve tissue could be spared, and an entirely competent valve achieved, which then would be two-thirds native tissue. The pericardial leaflet tissue could be treated with contemporary techniques for preventing calcification, but if the artificial leaflet became immobile late postoperatively, it still could act as a coaptation baffle for the other leaflets, and possibly not require additional operations, as can occur with total heterograft replacement.

The following examples are included to demonstrate preferred embodiments of the invention. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing from the spirit and scope of the invention. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein, will become apparent to those skilled in the art from the foregoing description. Such modifications are intended to fall within the scope of the appended claims.

### EXAMPLE 1

The inventors obtained and analyzed high resolution multi-slice computer tomography (CT) angiograms of healthy human aortic valves. Using 1 mm axial slices, CT angiograms from 10 normal aortic roots generated x, y, and z coordinates of valve structures in Mathematica. Three-dimensional least squares regression of leaflet and sinus coordinates was employed to compare hemispherical and ellipsoidal models. Shapes and dimensions of all root structures were evaluated.

Normal valve geometry could be represented as three hemispheres nested within a cylinder. However, dimensional fits were better using ellipsoidal geometry, with taller leaflets than predicted by hemispheres. Leaflet/sinus horizontal circumference was fairly circular (average minor-major ratio = 0.82-0.87). The base of the valve was quite elliptical (minor-major ratio = 0.65), and this geometry extended vertically. The commissure between the left (LC) and non-coronary (NC) cusps was located at the posterior junction of the base minor diameter and circumference, with the center of the right coronary (RC) cusp opposite. Centrums of the LC, NC, and RC leaflet/sinus ellipsoids were migrated toward the center of the valve (average fractional migration or alpha = 0.24, 0.32, and 0.09, respectively). The commissures flared outward by 5-10 degrees, and the RC cusp was the largest (Table 1).

**Table 1**

| Structure | Base | NC | RC | LC |
|---|---|---|---|---|
| Circumference | 73.7 mm | --- | --- | --- |
| Leaflet Height | --- | 12.7 mm | 12.9 mm | 12.5 mm |
| Major Axis | 14.1 mm | 8.6 mm | 9.5 mm | 8.7 mm |
| Minor Axis | 9.1 mm | 7.4 mm | 7.8 mm | 7.6 mm |
| Minor/Major Axis Ratio | 0.65 | 0.86 | 0.82 | 0.87 |
| Alpha | --- | 0.32 | 0.09 | 0.24 |
| Leaflet Area | --- | 616 mm² | 670 mm² | 620 mm² |
| Leaflet Volume | --- | 1959 mm³ | 2238 mm³ | 1998 mm³ |

Furthermore, examination of the sub-commissural regions showed that the commissures flared outward from the center of the valve by about 5 to 10 degrees. Therefore, mounting frames were designed with an elliptical cross section with narrowed and flared posts, as described herein and below.

### EXAMPLE 2

The currently described intra-annular mounting frame with an elliptical cross section of 1.5 axis ratio (major axis/minor axis, or 0.66 axis ratio minor axis/major axis) and 10° outward post flaring was tested in calves with promising results, as detailed below.

Mounting frames were implanted into 10 calves for survival studies at the Texas Heart Institute. Calves were used as the implant model because calves have valves that are near-human size, providing good correspondence to the devices that will be used clinically in humans. Echocardiography analysis of the repaired valve showed good leaflet coaptation, normal leaflet opening, no leaks, and undisturbed laminar flow. CT-angiography showed normal elliptical leaflet geometry and coaptation during diastole, and good opening of the valve leaflets during systole. These findings were confirmed using root angiography.

At autopsy, endoscopy was performed under water pressurization, and it was shown in all of the calves that the leaflets were nicely aligned with good verticality and no coaptation problems - they meet in the midline, and the pledgets are well-endothelialized.

The presently described mounting frame is currently scheduled to undergo clinical testing.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the scope of the invention as defined by the appended claims.

## Claims

1. An intra-annular mounting frame (10) for an aortic valve comprising:
a) a plurality of curvatures (12) having ends;
b) a plurality of points (14) interconnecting the ends of each of the curvatures to form an interior area; and
c) a plurality of posts (16) extending upward from each of the points;
**characterised in that** the points, the ends of the curvatures, and the posts define edge regions (18) that flare outwardly from the interior area of the intra-annular mounting frame at an angle of from about 5 degrees to about 15 degrees measured from a vertical plane of the intra-annular mounting frame, wherein the intra-annular mounting frame has an elliptical shape having a major axis (D) and a minor axis (A), and wherein the ratio of the major axis to the minor axis is between about 1.1 to about 1.8.

2. The intra-annular mounting frame of claim 1, wherein the intra-annular mounting frame comprises three curvatures, three points, and three posts.

3. The intra-annular mounting frame of claim 1, wherein the intra-annular mounting frame comprises two curvatures, two points, and two posts.

4. The intra-annular mounting frame of claim 1, wherein the edge regions flare outward from the interior area of the intra-annular mounting frame at an angle of about 10 degrees measured from the vertical plane of the intra-annular mounting frame.

5. The intra-annular mounting frame of claim 1, wherein the ratio of the major axis (D) of the ellipse to the minor axis (A) of the ellipse is about 1.5.

6. The intra-annular mounting frame of claim 1, wherein the length of the major axis (D) of the ellipse is between about 10 millimeters and about 35 millimeters.

7. The intra-annular mounting frame of claim 1, wherein the length of the minor axis (A) of the ellipse is between about 8 millimeters and about 25 millimeters.

8. The intra-annular mounting frame of claim 1, wherein the intra-annular mounting frame comprises a plastic, a polymer, a metal, a thermoplastic, a resin, or combinations thereof.

9. The intra-annular mounting frame of claim 8, wherein the intra-annular mounting frame comprises metal.

10. The intra-annular mounting frame of claim 9, wherein the intra-annular mounting frame comprises metal wire.

11. The intra-annular mounting frame of claim 1, wherein the intra-annular mounting frame is coated or covered.

12. The intra-annular mounting frame of claim 11, wherein the intra-annular mounting frame is coated or covered with a polymer-type fiber cloth.

## Patentansprüche

1. Ringinterner Montagerahmen (10) für eine Aortenklappe, umfassend:
a) eine Mehrzahl von Krümmungen (12) mit Enden;
b) eine Mehrzahl von Punkten (14), die die Enden jeder der Krümmungen verbinden, um einen Innenbereich zu bilden;
c) und eine Mehrzahl von Stäben (16), die sich von jedem der Punkte nach oben erstrecken;
**dadurch gekennzeichnet, dass** die Punkte, die Enden der Krümmungen und die Stäbe Kantenbereiche (18) definieren, die sich von dem Innenbereich des ringinternen Montagerahmens in einem von einer vertikalen Ebene des ringinternen Montagerahmens gemessenen Winkel von etwa 5 Grad bis etwa 15 Grad nach außen aufweiten, wobei der ringinterne Montagerahmen eine elliptische Form hat, mit einer Hauptachse (D) und einer Nebenachse (A), und wobei das Verhältnis der Hauptachse zur Nebenachse zwischen etwa 1,1 bis etwa 1, 8 liegt.

2. Ringinterner Montagerahmen nach Anspruch 1, wobei der ringinterne Montagerahmen drei Krümmungen, drei Punkte und drei Stäbe hat.

3. Ringinterner Montagerahmen nach Anspruch 1, wobei der ringinterne Montagerahmen zwei Krümmungen, zwei Punkte und zwei Stäbe hat.

4. Ringinterner Montagerahmen nach Anspruch 1, wobei sich die Kantenbereiche von dem Innenbereich des ringinternen Montagerahmens in einem von der vertikalen Ebene des ringinternen Montagerahmens gemessenen Winkel von etwa 10 Grad nach außen aufweiten.

5. Ringinterner Montagerahmen nach Anspruch 1, wobei das Verhältnis der Hauptachse (D) der Ellipse zur Nebenachse (A) der Ellipse etwa 1,5 beträgt.

6. Ringinterner Montagerahmen nach Anspruch 1, wobei die Länge der Hauptachse (D) der Ellipse zwischen etwa 10 Millimeter und etwa 35 Millimeter liegt.

7. Ringinterner Montagerahmen nach Anspruch 1, wobei die Länge der Nebenachse (A) der Ellipse zwischen etwa 8 Millimeter und etwa 25 Millimeter liegt.

8. Ringinterner Montagerahmen nach Anspruch 1, wobei der ringinterne Montagerahmen einen Kunststoff, ein Polymer, ein Metall, einen Thermoplast, ein Harz oder Kombinationen davon umfasst.

9. Ringinterner Montagerahmen nach Anspruch 8, wobei der ringinterne Montagerahmen Metall umfasst.

10. Ringinterner Montagerahmen nach Anspruch 9, wobei der ringinterne Montagerahmen Metalldraht umfasst.

11. Ringinterner Montagerahmen nach Anspruch 1, wobei der ringinterne Montagerahmen beschichtet oder überzogen ist.

12. Ringinterner Montagerahmen nach Anspruch 11, wobei der ringinterne Montagerahmen mit einem Fasertuch vom Polymertyp beschichtet oder überzogen ist.

## Revendications

1. Cadre de montage intra-annulaire (10) destiné à une valvule aortique comprenant :
a) un ensemble de parties courbes (12), ayant des extrémités,
b) un ensemble de points (14) reliant les extrémités de chacune des parties courbes pour former une zone interne, et
c) un ensemble de colonnettes (16) s'étendant vers le haut à partir de chaque point,
**caractérisé en ce que** les points, les extrémités des parties courbes et les colonnettes définissent des régions de bord (18) qui s'évasent vers l'extérieur à partir de la zone interne du cadre de montage intra-annulaire selon un angle d'environ 5 degrés à environ 15 degrés, mesuré à partir d'un plan vertical du cadre de montage intra-annulaire, le cadre de montage intra-annulaire ayant une forme elliptique ayant un grand axe (D) et un petit axe (A), le rapport du grand axe au petit axe étant compris entre environ 1,1 et environ 1,8.

2. Cadre de montage intra-annulaire conforme à la revendication 1, comprenant trois parties courbes, trois points et trois colonnettes.

3. Cadre de montage intra-annulaire conforme à la revendication 1, comprenant deux parties courbes, deux points et deux colonnettes.

4. Cadre de montage intra-annulaire conforme à la revendication 1, dans lequel les régions de bord s'évasent vers l'extérieur à partir de la zone interne du cadre de montage intra-annulaire selon un angle d'environ 10 degrés mesuré à partir du plan vertical du cadre de montage intra-annulaire.

5. Cadre de montage intra-annulaire conforme à la revendication 1, dans lequel le rapport du grand axe (D) de l'ellipse au petit axe (A) de l'ellipse est égal à environ 1,5.

6. Cadre de montage intra-annulaire conforme à la revendication 1, dans lequel la longueur du grand axe (D) de l'ellipse est comprise entre environ 10 millimètres et environ 35 millimètres.

7. Cadre de montage intra-annulaire conforme à la revendication 1, dans lequel la longueur du petit axe (A) de l'ellipse est comprise entre environ 8 millimètres et environ 25 millimètres.

8. Cadre de montage intra-annulaire conforme à la revendication 1, renfermant une matière plastique, un polymère, un métal, un matériau thermoplastique, une résine ou des combinaisons de ces éléments.

9. Cadre de montage intra-annulaire conforme à la revendication 8, renfermant du métal.

10. Cadre de montage intra-annulaire conforme à la revendication 9, renfermant un fil métallique

11. Cadre de montage intra-annulaire conforme à la revendication 1, revêtu ou recouvert.

12. Cadre de montage intra-annulaire conforme à la revendication 11, revêtu ou recouvert d'un tissu de fibres de type polymère.
